# EUROPEAN PATENT SPECIFICATION

(11) **EP 0 709 136 B1**
(45) Date of publication and mention of the grant of the patent: **13.10.1999**
(21) Application number: 94921090.0
(22) Date of filing: 13.07.1994
(51) Int. Cl.: B02C 19/20, G01N 1/28, C12M 1/33

(54) **BAG FOR CRUSHING AND DISPERSION**
BEUTEL ZUM ZERKLEINERN UND DISPERGIEREN VON PROBEN
SAC D'ECRASEMENT ET DE DISPERSION

(30) Priority: 14.07.1993 JP 19686393
(43) Date of publication of application: 01.05.1996
(73) Proprietor: SHIMAKYU CHEMICAL CO., LTD., Osaka-shi, Osaka 532 (JP)
(72) Inventor: TAKEMOTO, Masahiro Shimakyu Chemical Co., Ltd., Yodogawa-ku Osaka-shi Osaka 532 (JP); YAMAUCHI, Hiroshige Shimakyu Chemical Co., Ltd., Osaka-shi Osaka 532 (JP)
(74) Representative: Vetter, Hans, Dipl.-Phys. Dr.
(86) International application number: JP9401152
(87) International publication number: WO9502458

(56) References cited:
- JP-A- 5 187 977
- JP-U- 1 041 300

## Description

### TECHNICAL FIELD

This invention relates to a crushing, comminuting and dispersing bag for mechanically crushing, comminuting and dispersing foodstuffs, soil or other samples.

### BACKGROUND ART

In microbiological examination or physiological examination, foodstuffs, soil or other samples are crushed, comminuted and dispersed in solvents or other to emulsify. Conventionally, for this purpose, a mortar and a pestle is used, or the sample is introduced into a cup with dispersion medium and then crushed, comminuted and dispersed by rotating a cutter like a screw. Such device is a so-called cup type homogenizer. For the sake of microbiological examinations, a device (the trade name Stomacher: generally distributed by Gunze Sangyo Kabushiki Kaisha) is provided. In operation of the device, a polyethylene bag with the sample and the dispersion medium sealed therein is compressed alternatively by a pair of paddles (see JP-U-64-41300). By the pulsatile pressure applied to the bag, microorganisms are extracted from the sample and dispersed.

However, when the mortar and the pestle or the cup type homogenizer are used, they need to be cleaned or sterilized before usage. Therefore, when a number of samples are crushed, comminuted and dispersed, a number of mortars and pestles or a number of cups and cutters need to be prepared, which is troublesome. Moreover, since it is difficult to operate the mortar and the pestle in aseptic environment, they are not suitable for microbiological examinations.

Since a disposable polyethylene bag is used for Stomacher, a number of samples can be efficiently crushed, comminuted and dispersed. However, Stomacher is too large to be transported. Furthermore, Stomacher is inappropriate for effectively extracting and dispersing microorganisms existing in octopus, squid or other elastic samples because the samples are crushed, comminuted and dispersed by the pulsatile pressure applied to the dispersion medium.

### DISCLOSURE OF THE INVENTION

Wherefore, the object of the present invention is to easily, efficiently and effectively crush, comminute and disperse various samples without using any expensive device.

To attain the aforementioned object, the invention provides a crushing, comminuting and dispersing bag characterized in that a synthetic resin thin flat sheet with file-like unevenness or irregularities on at least one surface is disposed inside a synthetic resin bag member, and a sealing member is provided for sealing the opening of said synthetic resin bag member.

In operation of the aforementioned structure of the invention, the sample and the dispersion medium are introduced into the synthetic resin bag member and the opening of the bag member is sealed with the sealing member. Then, by squeezing the bag member with a rubber roller or other device, the sample is crushed or comminuted by the file-like irregularities provided on the surface of the thin flat sheet disposed inside the bag member, and homogeneously dispersed in the dispersion medium.

The present invention requires no expensive devices. Just by squeezing the bag member with a rubber roller or other device, the sample is easily crushed, comminuted and dispersed. Further in the present invention, the sample rubs against the irregularities and is thus mechanically ground. Therefore, even octopus, squid or other resilient samples can be effectively crushed, comminuted and dispersed.

Additionally, the present invention has a simple structure and is inexpensively manufactured of synthetic resin or other material. Further, the bag according to the present invention can be easily transported and stored. With a number of disposable bags, a number of samples can be efficiently crushed, comminuted and dispersed.

### BRIEF EXPLANATION OF DRAWING FIGURES

Fig. 1 is a plan view showing the structure of a crushing, comminuting and dispersing bag of Embodiment 1 of the present invention, and Fig. 2 is a cross-sectional view of a crushing, comminuting and dispersing bag shown in Fig. 1.

Fig. 3 is a plan view showing the structure of a crushing, comminuting and dispersing bag of Embodiment 2 of the present invention, and Fig. 4 is a cross-sectional view of a crushing, comminuting and dispersing bag shown in Fig. 3.

### BEST MODE FOR CARRYING OUT THE INVENTION

The embodiment of the invention is now explained referring to the drawing figures.

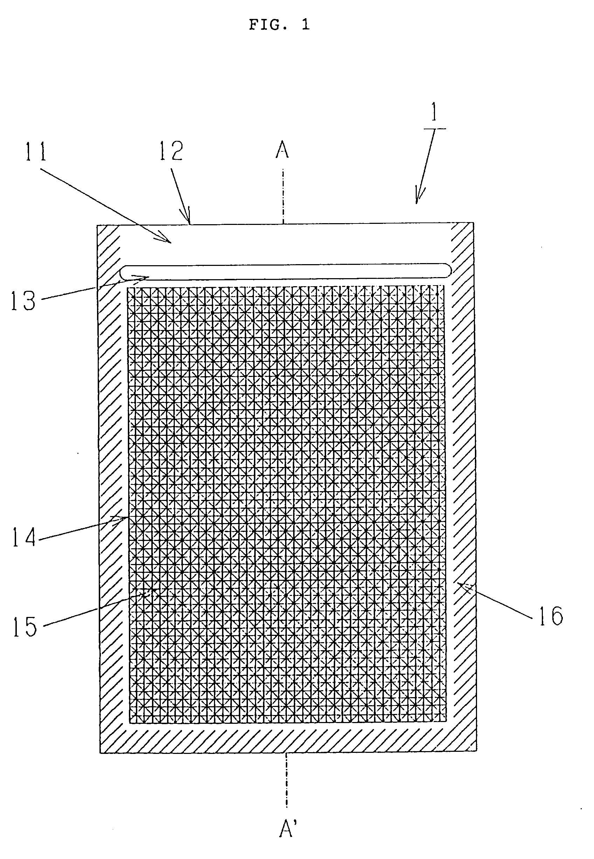

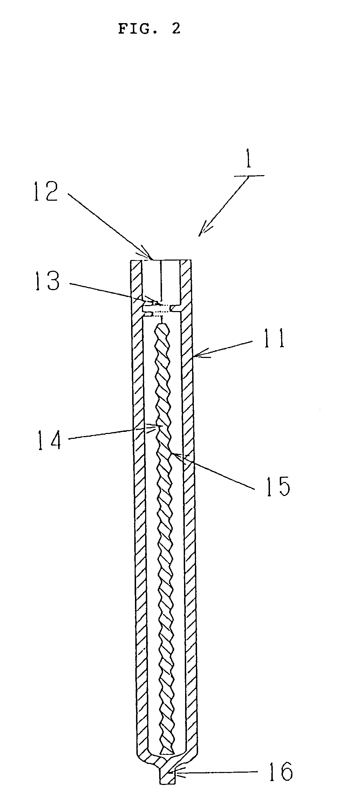

Fig. 1 is a plan view showing the structure of a crushing, comminuting and dispersing bag 1 of Embodiment 1 of the present invention. Fig. 2 is a cross-sectional view taken along line A-A' of Fig. 1.

As shown in Fig. 1, the crushing, comminuting and dispersing bag 1 is composed of a rectangular synthetic resin bag member 11 with a thin synthetic resin flat sheet 14 disposed therein. The bag member 11 is formed by welding two sheets of synthetic resin film at welding portion 16 on three of the side edges. The length of the thin flat sheet 14 is slightly shorter than the effective inside dimension of the bag member excepting the length between an opening 12 and a sealing member 13. On both surfaces of the thin flat sheet 14, numbers of small four-cornered pyramidal projections 15 are provided to form file-like irregularities. The sealing member 13 has a conventional structure, a fastener, in which the opening is sealed by engaging a groove with a ridge.

In operation of the crushing, comminuting and dispersing bag 1 having the aforementioned structure, the sample and the dispersion medium are introduced into the bag through the opening 12, which is then sealed with the fastener 13. Consequently, the crushing, comminuting and dispersing bag 1 is put on a solid flat table or other and squeezed with a rubber roller or other device. Thus, the sample is crushed and comminuted by the file-like irregularities formed on the surface of the thin flat sheet 14 inside the bag member, and is homogeneously dispersed in the dispersion medium. The file-like irregularities are preferably formed on both surfaces of the thin flat sheet 14 so that the sample may be introduced into both sides of the thin flat sheet 14.

The size of the synthetic resin bag member 11 is not especially limited. When 5g to 10g of the sample is used and the dispersion medium has the amount 10 times larger than the sample, the bag member preferably has a width of 6cm to 15cm and a length of 10cm to 25cm for easy operation. The thickness of the synthetic resin film composing the bag member is not especially limited and varies with the material of the film. The film can have any thickness, so long as the bag is soft and resilient enough to be squeezed with a rubber roller or other device and sufficiently strong to resist tearing. The preferable thickness of a polyethylene film, for example, is about 0.04mm to 0.12mm.

The thickness of the bottom portion of the synthetic resin flat sheet 14 disposed inside the synthetic resin bag member 11, excepting the height of the four-cornered pyramidal projections, is not especially limited. The sheet can have any thickness, so long as the bag is easily operated and sufficiently strong. The preferable thickness of a polyethylene flat sheet, for example, is about 0.5mm to 3mm. Additionally, the size of the file-like irregularities 15 is not especially limited. The four-cornered pyramidal projections preferably have length of one side of the bottom about 2mm to 4mm and height about 1mm to 3mm for grinding and comminuting the samples efficiently.

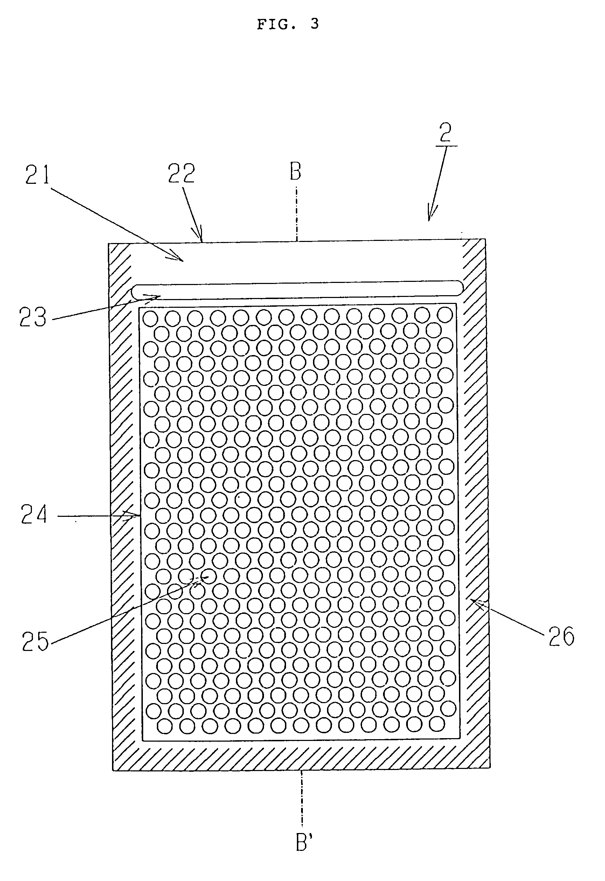

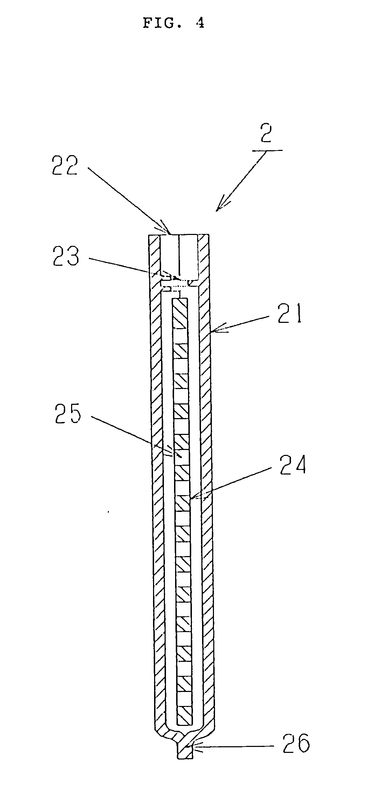

Fig. 3 is a plan view showing the structure of a crushing, comminuting and dispersing bag 2 of Embodiment 2 of the present invention, and Fig. 4 is a cross-sectional view taken along line B-B' of Fig. 3.

As shown in Fig. 3, the crushing, comminuting and dispersing bag 2 is composed of a rectangular synthetic resin bag member 21 with a thin synthetic resin flat sheet 24 disposed therein. The bag member 21 is formed by welding two sheets of synthetic resin film at welding portion 26 on three of the side edges. Numbers of small holes 25 are formed through the thin flat sheet 24. The length of the thin flat sheet 24 is slightly shorter than the effective inside dimension of the bag member. A sealing member 23 having a conventional structure, a fastener, is provided for sealing the opening by engaging a groove with a ridge.

In operation of the crushing, comminuting and dispersing bag 2 having the aforementioned structure, the sample and the dispersion medium are introduced into the bag through the opening 22, which is then sealed with the fastener 23. Consequently, the crushing, comminuting and dispersing bag 2 is put on a solid flat table or other and squeezed with a rubber roller or other device. Thus, the sample is crushed, comminuted and dispersed homogeneously in the dispersion medium by the file-like irregularities formed around the edges of the small holes 25 through the flat sheet 24 inside the bag member. As the small holes 25 are formed through the thin flat sheet 24 disposed inside the bag member, the sample may be introduced into both sides of the thin flat sheet 24.

The size of the synthetic resin bag member 21 composing the crushing, comminuting and dispersing bag is not especially limited. When 5g to 10g of the sample is used and the dispersion medium has the amount 10 times larger than the sample, the bag member preferably has the width of 6cm to 15cm and a length of 10cm to 25cm for easy operation. The thickness of the synthetic resin film composing the bag member is not especially limited and varies with the material of the film. The film can have any thickness, so long as the bag is soft and resilient enough to be squeezed with a rubber roller or other device and sufficiently strong to resist tearing. The preferable thickness of a polyethylene film, for example, is about 0.04mm to 0.12mm.

The thickness of the synthetic resin flat sheet 24 disposed inside the synthetic resin bag member 21 is not especially limited. The sheet can have any thickness, so long as the bag is easily operated and sufficiently strong. The preferable thickness of a polyethylene flat sheet, for example, is about 0.5mm to 3mm. Additionally, for grinding and comminuting the sample sufficiently, the dimension of the round small holes formed through the flat sheet 24 about 2mm to 6mm and the distance between the holes about 1mm to 3mm are preferable.

The present invention is not limited to the aforementioned embodiments. In the Embodiments 1 and 2, the sealing members 13 and 23 are provided integrally at the openings 12 and 22 of the bag members 1 and 2. However, the sealing members 13 and 23 may be provided independently from the bag members 1 and 2. For example, clips may be applied to seal the openings 12 and 22.

In the Embodiment 1, numbers of four-cornered pyramidal projections are provided to form irregularities on the surface of the thin flat sheet 14 inside the bag member. However, the projections may have any other configurations. For example, three-cornered pyramidal or conical projections may be provided. In the Embodiment 2, round small holes are formed through the thin flat sheet 24 inside the bag member. However, the holes may have any other configurations. For example, square or triangular small holes may be formed.

Furthermore, in the Embodiments 1 and 2, the bag member is squeezed with a hand-operated rubber roller. However, the way to apply pressure from the outside of the bag member to the sample is not especially limited. For example, the bag member may be squeezed by hand for crushing, comminuting and dispersing the sample.

### EXPERIMENTAL EXAMPLES

The experimental examples are hereinafter described for proving the effectiveness of the above mentioned embodiments. The inventors of the present invention conducted the experiment of the so-called homogenizing by using the crushing, comminuting and dispersing bag 2 of the Embodiment 2. The crushing, comminuting and dispersing bag 2 was composed of a polyethylene bag member 2 having a thickness of 0.08mm, a width of 120mm, a length of 180mm and a width of the welding portion of 7mm, and a polyethylene thin flat sheet 24 having the thickness of 0.5mm, the length of 160mm and the width of 100mm. Through the thin flat sheet 24 was provided numbers of small holes 25 having 5.0mm of dimension and spaced at every 7.0mm. In the experiment, using the aforementioned crushing, comminuting and dispersing bag 2, the samples were crushed, comminuted and dispersed to emulsify as follows.

First, numbers of the crushing, comminuting and dispersing bags 2 were sterilized with ethylene oxide gas. After each of the samples given in Table 1 was cut into pieces, 5.0g each of the samples was introduced into each of the crushing, comminuting and dispersing bags 2. Subsequently, after 45.0ml of physiological salt solution was each injected to the crushing, comminuting and dispersing bag 2, the bag 2 was sealed with the fastener 23. Then the crushing, comminuting and dispersing bag 2 was put on an experiment table and was squeezed for 1 to 2 minutes with a hand-operated rubber roller which is generally used for pasting up wallpapers or for other purposes. While the crushing, comminuting and dispersing bag 2 was squeezed, the degree of comminution of the sample was visually examined and the portions of the sample having a low degree of comminution were mainly squeezed.

For reference examples, the same samples as those used in the embodiment were homogenized as follows.

### Reference Example 1

10.0g each of the samples was introduced into each of thermally sterilized homogenizer cups. After 90ml each of sterilized physiological salt solution was injected into the cup, the cutter was rotated at 10,000 rpm so as to homogenize the sample for about 1 minute. Homogenizer AM-12 (the trade name: manufactured by Kabushiki Kaisha Nihon Seiki Seisakusho) was used as the cup type homogenizer.

### Reference Example 2

10.0g each of the samples was introduced into each of gamma-ray sterilized polyethylene bags for Stomacher. After 90ml each of sterilized physiological salt solution was injected into the bag and the bag was sealed, the sample was homogenized, i.e. stomached for about 1 minute. STOMACHER 400 (the trade name: generally distributed by Gunze Sangyo Kabushiki Kaisha) was used.

The number of microorganisms existed in the samples homogenized in respective ways were measured with a conventional method, and the extracting efficiency of gained microorganisms was examined. The number and the extracting efficiency of microorganisms is shown in Table 1.

**Table 1**

| SAMPLES | EMBODIMENT | REFERENCE 1 | REFERENCE 2 |
|---|---|---|---|
| FROZEN SHRIMP | 1,150,000 (94.3) | 1,220,000 (100.0) | 979,000 (80.2) |
| RAW SQUID | 470,000 (104.4) | 450,000 (100.0) | 380,000 (84.4) |
| BOILED OCTOPUS | 20,000 (105.2) | 19,000 (100.0) | 17,000 (89.4) |
| SALTED CHINESE CABBAGE | 2,000,000 (97.6) | 2,050,000 (100.0) | 2,000,000 (97.6) |
| BOILED CONGER | 42,000 (100.0) | 42,000 (100.0) | 45,000 (107.1) |
| THE UPPER NUMERAL VALUE: THE NUMBER OF GENERAL BACTERIA PER 1g OF SAMPLE THE LOWER NUMERAL VALUE WITHIN THE PARENTHESIS: EXTRACTING EFFICIENCY, WHEN THE EFFICIENCY OF THE HOMOGENIZING METHOD OF REFERENCE EXAMPLE 1 IS SET AS 100% | | | |

As shown in Table 1, in the Embodiment 2, the extracting efficiency almost the same as that of the homogenizing method using the cup-type homogenizer according to the Reference Example 1 can be obtained. Therefore, in the Embodiment 2 any sample can be homogenized sufficiently. Especially regarding squid and octopus, the Embodiment 2 shows the extracting efficiency superior to that of the stomaching method of Reference 2. Since the sample rubs against the irregularities formed on the surface of thin flat sheet 24 in the Embodiment 2, even resilient materials can be efficiently crushed, comminuted and dispersed.

As aforementioned, the crushing, comminuting and dispersing bag 2 of the Embodiment 2 requires no expensive devices. By squeezing the crushing, comminuting and dispersing bag 2 with a hand-operated rubber roller, the sample can be easily crushed, comminuted and dispersed to emulsify. The effectiveness of the crushing, comminuting and dispersing bag 1 of the Embodiment 1 can be proved in the same manner with the Embodiment 2.

### INDUSTRIAL APPLICABILITY

As detailed above, the crushing, comminuting and dispersing bag of the present invention requires no expensive devices. By squeezing the bag member with a hand-operated rubber roller or other device, the sample can be easily crushed, comminuted and dispersed. Additionally, in the present invention, as the sample rubs against the irregularities formed on the surface of the thin flat sheet, even resilient material can be efficiently crushed, comminuted and dispersed.

Furthermore, the bag according to the present invention can be inexpensively manufactured of synthetic resin or other material. The bag can be easily transported and stored as well. With a number of disposable bags, many samples can be efficiently crushed, comminuted and dispersed.

## Claims

1. A crushing, comminuting and dispersing bag (1) having:
a synthetic resin bag member (11), in which a thin flat sheet (14) with file-like irregularities on at least one surface thereof is disposed; and
a sealing member (13) for sealing the opening (12) of the bag member.

2. A crushing, comminuting and dispersing bag according to claim 1, in which the irregularities (15) are formed by providing numbers of small projections on the surface of the thin flat sheet.

3. A crushing, comminuting and dispersing bag according to claim 1, in which the irregularities (15) are formed by providing numbers of small holes through the thin flat sheet.

## Patentansprüche

1. Zerkleinerungs-, Zerreibungs- und Dispergierungsbeutel (1) mit:
einem Kunstharz-Beutelelement (11), in welchem eine dünne flache Lage (14) mit feilenartigen Unregelmäßigkeiten an mindestens einer ihrer Flächen angeordnet ist; und
einem Abdichtungselement (13) zum Abdichten der Öffnung (12) des Beutelelements.

2. Zerkleinerungs-, Zerreibungs- und Dispergierungsbeutel nach Anspruch 1, in welchem die Unregelmäßigkeiten (15) durch Bereitstellen zahlreicher kleiner Erhebungen auf der Fläche der dünnen flachen Lage gebildet sind.

3. Zerkleinerungs-, Zerreibungs- und Dispergierungsbeutel nach Anspruch 1, in welchem die Unregelmäßigkeiten (15) durch Bereitstellen zahlreicher kleiner Löcher durch die dünne flache Lage hindurch gebildet sind.

## Revendications

1. Poche de broyage, pulvérisation et dispersion (1) ayant :
un élément (11) formant poche en résine synthétique, dans lequel est disposée une feuille mince et plate (14) pourvue d'irrégularités semblables à celles d'une lime sur au moins une de ses surfaces ; et
un élément d'étanchéité (13) pour fermer hermétiquement l'ouverture (12) de l'élément formant poche.

2. Poche de broyage, pulvérisation et dispersion selon la revendication 1, dans laquelle les irrégularités (15) sont formées en ménageant de nombreuses petites saillies sur la surface de la feuille mince et plate.

3. Poche de broyage, pulvérisation et dispersion selon la revendication 1, dans laquelle les irrégularités (15) sont formées en ménageant de nombreux petits trous dans la feuille mince et plate.
